# EUROPEAN PATENT APPLICATION

(11) **EP 3 156 373 A1**
(43) Date of publication of application: **19.04.2017**
(21) Application number: 15807281.9
(22) Date of filing: 15.06.2015
(51) Int. Cl.: C02F 3/30, C02F 11/02

(54) **EQUIPMENT AND METHOD FOR MODULATING MICROBIAL CONSORTIA**

(30) Priority: 13.06.2014 BR 102014014539
(71) Applicant: Biotecam Assessoria e Desenvolvimento de Tecnologia Ambiental Ltda., 21941-614 Rio de Janeiro (BR)
(72) Inventor: REMER, Ricardo Amaral, 22471-001 Rio de Janeiro (BR); DEGRAVE, Wim Maurits Sylvain, 22241-125 Rio de Janeiro (BR)
(74) Representative: Trillat, Anne-Cecile
(86) International application number: PCT/BR2015/050076
(87) International publication number: WO 2015/188245

(57) **Abstract**

The present invention refers to the fields of Environmental Engineering and Microbiology, being related to Energy Conservation and Waste Management. More specifically, the present invention provides an equipment and a method for *on-site* and *ex-situ* modulation of microbial consortia useful in liquid and semi-solid effluents or solid waste treatment systems, for removing and/or reusing undesirable organic or inorganic loads. The equipment of the invention consists of a main cultivation tank or a liquid body provided with an *ex-situ* subsystem for modulating microbial consortia for subsequent or concomitant introduction into the main cultivation tank or reactor or into the liquid body. In one embodiment, the *on-site* and *ex-situ* process of the invention is implemented in a culture equipment with controlled conditions for introducing the microorganism-enriched material into the effluent or solid waste to be treated, in the form of pre-cultivation and/or co-cultivation. In another embodiment, the *ex-situ* subsystem of the invention comprises a device for forming gas/air bubbles in a liquid suitable for applying a shearing force to the microorganisms present in the subsystem, and also useful for modulating the microbial consortia in a main cultivation tank or a liquid body. The invention substantially enhances the performance of effluent treatment systems, increasing the capacity of already installed units and/or reducing the cost of installation of new units; it allows the use of effluents as biomass for the biotransformation and generation of products of economic interest.

## Description

### Field of the Invention

The industrial creation described herein comprises an invention capable of patent protection. Said invention is in the fields of Environmental Engineering and Microbiology, being related to Energy Conservation and Waste Management. More specifically, the present invention provides equipment and an *on-site* and *ex-situ* process of microbial consortia modulation useful in treatment systems of semi-solid, liquid effluents or solid waste, for the removal and/or reuse of undesirable organic or inorganic loads. The equipment of the invention consists of a main cultivation tank or a liquid body equipped with *ex-situ* subsystem of microbial consortia modulation, for subsequent or concomitant introduction of all or part of its volume to the reactor/main cultivation tank or to the liquid body. In one embodiment, the *on-site* and *ex-situ* process of the invention is conducted in equipment of culture with controlled conditions, for introducing the material enriched with microorganisms, or components thereof, to the effluent or solid waste to be treated, in the form of pre-cultivation and/or co-cultivation. In another embodiment, the *ex-situ* subsystem of the invention comprises a device for forming gas/air bubbles in a liquid adjusted to provide shearing microorganisms present in the subsystem, being additionally useful in the microbial consortia modulation of a main cultivation tank or a liquid body.

### Background of the Invention

Effluent treatment systems are so called because of the premise related to their emergence and development. The premise is that effluents must be "treated" so that undesirable substances are removed from them. The "treated" liquid body is the product of said systems and the organic load and other undesirable substances are byproducts. In this context, several effluent treatment processes are available, being able to divide such processes between aerobic processes and anaerobic processes.

The biological treatment systems of liquid effluents or solid or semi-solid waste have the capabilities or "abilities" of microorganisms to degrade undesirable substances. In such systems, the control of the ecosystem offered to the microorganisms is of vital importance in several aspects, such as the efficiency and productivity of treatment systems, being relevant, to mention just a few aspects, the temperature, the pH, the presence of gases dissolved in the liquid body, the presence and concentration of substances which may be toxic to the microorganisms or difficult to degrade by them. However, in treatment systems of liquid, solid or semi-solid waste, ideal conditions for the growth of microorganisms that act in the degradation of undesirable substances are not always available.

The biological systems of the treatment of liquid effluents are generally sized to operate in continuous or semi-continuous regime, due to the high volume. However, continuous or semi-continuous regimes of biological systems suffer with the variations in the environmental conditions, with the variation of concentration of substances to be treated, with the occurrence of contamination with undesirable organisms, the occurrence of phage lysis, among others. Such variations, which are typical in effluent and sewage treatment plants, greatly affect the efficiency of treatment systems, which for these reasons generally require a larger dimensioning. The present invention provides a solution to these problems.

Different approaches to the performance improvement of treatment systems of solid, semi-solid and liquid waste exist in the market and in the prior art, including the addition of microorganisms as inputs purchased in bottles or sacks. Such approaches aim to remedy or compensate for the effects of typical instabilities of treatment systems and generally act in a way to significantly increase the efficiency of the treatment system. In this context, products for increasing the titers of microorganisms in aerobic treatment stations of liquid effluents are available in the market. The common practice is the acquisition of microorganisms previously cultured and added as powder containing great amount of spores or lyophilized microorganisms, being useful in the form of re-composition of specific microbial flora, after detection of low titers of microorganisms or upon detection of substances which are preferably degraded by the microorganisms to be added. However, this practice faces considerable technical problems: The cost and availability of the microorganisms, which are generally imported and suffer from the exchange variation; the logistical difficulties of transport and stock; environmental and/or human health risks, because it is the transport of microbial material in great amount; and, particularly, the imitation of quantity available for introducing in treatment systems and the low state of metabolic activation of organisms when introduced under these conditions. Additionally, a critical problem often precludes the treatment of water courses, such as lakes, ponds, rivers, canals, arms of rivers or the sea, bays, etc., due to the prohibition of the introduction of exogenous microorganisms into such water courses, for its possible and/or unknown environmental impact. The present invention also provides a solution to these problems.

Another significant difficulty in the prior art concerning the aerobic systems of biological treatment lies in the fact that the vast majority of such treatment systems require a lot of energy for the introduction of air and/or its dissolution into the liquid body. The low solubility of oxygen in water is known, notably at elevated temperatures typical of tropical regions. Thus, the growth and obtaining of high titers of microorganisms in reactors/effluent treatment tanks has been a great technical challenge, once the growth of organisms under such conditions is predominantly limited by the low amount of oxygen dissolved in the liquid body. The present invention also provides a solution to these problems.

Still another significant difficulty in the prior art concerning the aerobic systems of biological treatment is, paradoxically, the great amount of present or produced microorganisms. Modulating the microbial consortia, both selectively and by fully eliminating live microorganisms, without thereby using antibiotics or other environmentally and economically unacceptable substances - is still a technical challenge not satisfactorily solved in the art, notably the volumes associated with effluent treatments or in the recovery of liquid bodies such as rivers, ponds, lagoons and coves. The present invention also provides a solution to these problems.

The searches in the patent literature pointed to some partially relevant documents in the context of the present invention, which will be described in chronological order, in order to facilitate the perception of the different paths followed by the technique until then - and the lack of convergence of known technologies, when compared to the present invention.

The document US 4,556,491, entitled "*Avoidance of rising sludge in biological wastewater treatment clarifiers*", was published in 03Dez1985. Said document reveals a method to avoid the increase of sludge in secondary clarifiers of biological treatment plants. The proposed solution involves minimizing the entry of nitrogen gas with the liquor entering the clarifier, so that the dissolved nitrogen level in the biomass decanted in the clarifier remains lower than that of the equilibrium. Said method is most effective when soluble nitrogen oxides are present and are reduced by biomass to elemental nitrogen. The excess of nitrogen can be removed by removing gas from the liquor from the effluent entering the clarifier, which is done by deepening and widening the usual tank, so that the flow rate of the downstream liquor is less than 0.4 feet per second and the depth is greater than 4 feet, thereby allowing the macrobubbles containing gas to rise to the surface, releasing the gas. The free liquor of macrobubbles is then passed through a zone of hydraulic pressure on the way to the clarifier, forming microbubbles that dissolve in the liquid.

The document US 7,270,751, entitled "*Method for Treating of Sewage Plant Sludges by a Fungal Process*", was published for the first time on 18Set2003 (WO 03/076351). Said document reveals a process for the treatment of domestic effluents which comprise the application of microfungi, which are continuously cultivated in parallel, being aerobic both the effluent treatment and the continuous parallel cultivation of microfungi. Said process specifies the conditions in which the microfungi are cultivated and used, also indicating the equipment and filter media by sludge membrane. Said document differs from the present invention by numerous technical features, mentioning, among others: in addition to being limited to the cultivation of micro-fungi and requiring filtration membranes, the equipment and process of said document do not comprise a device for generating microbubbles and/or thin films and/or shearing in the *ex-situ* subsystem and/or in the main tank and do not aim to modulate the microbial consortium in the treatment tank. Nor is said process and equipment related to the treatment of liquid bodies such as rivers, ponds, lagoons and coves.

The document US 7,879,593, entitled "*Fermentation Systems, Methods and Apparatus*", was published for the first time on 09Out2003 (US 2003/0190742 A1). Said document reveals a process for the treatment of domestic effluents comprising a fermentation subsystem for enrichment of microorganisms, which are cultivated on-site and in parallel, for selective enrichment of certain types of microorganisms at the expense of other. Said document differs from the present invention by numerous technical features, mentioning, among other: the equipment and the process of said document do not comprise a device of microbubbles generation and/or thin films and/or of shearing in the *ex-situ* subsystem and/or in the main tank. Nor are said process and equipment also related to the treatment of liquid bodies such as rivers, ponds, lagoons and coves.

The document US 6,773,592, entitled "*Systems and methods for treating waste water using an inoculum*", was published on 10Ago2004. Said document reveals a method for treating effluents/sewage and involves the treatment of the collection system before the effluent reaches the treatment unit. A key aspect of said method is the introduction of an inoculum of microorganisms selected in a quiescent zone of the collection network. The quiescent zone is where the effluent is significantly decelerated in the collection network and may be temporarily stationary, that is, a pumping station or low area in two sections of a duct. Microorganisms tend to multiply in these quiescent zones. The introduction of inoculum from a competitive crop to unwanted microorganisms (which degrade effluent degradation) improves the efficiency of the entire treatment system. Said method resembles the known approach of prior art of adding cultured off-site microorganisms.

The document US 2005/0279713, entitled "*System and method for dissolving gases in liquids*", was published on 22Dez2005. Said document reveals the equipment and method to dissolve gas in a liquid and comprises saturation tank and a source of pressurized gas connected to a gas area (*head space*) of the saturation tank. The saturation tank contemplates a pressurized tank containing at least an injector nozzle which allows the passage of liquid into the pressurized tank and an output to the liquid containing dissolved gas. Upon passage of liquid containing gas into a second fluid, the gas is released in the form of microbubbles. The microbubbles assist in the flocculation of suspended particles and promote the dissolution of the gas in the second fluid. The preferred gas is air, oxygen or ozone, being those applicable to use in the treatment of rivers, ponds and industrial facilities.

The document US 7,488,713, entitled "*Waste water treatment plant and method*", was published on 10Fev2009. Said document reveals a sewage treatment plant which includes a reactor or chamber (26) containing a plurality of sewage carriers (38). In the base of chamber (26) is a chamber (40) of biofilm collection. In a coaxial arrangement with the chamber (26), there is a hollow duct (30) with an air injection device (32) disposed on the bottom thereof. During the use, the sewage enters the treatment chamber through the entrance (28) and is forced to circulate around the treatment chamber through the action of duct pumping (30) and the air injection. The Treated water passes through the biofilm collection chamber (4) to a decantation chamber (42), which has an output (44) to the treated water.

The document WO 2012/065250, entitled "*Integrated dissolved air flotation system and process for the treatment of wastewater*", was published on 24Mai2012. Said document reveals a method useful for the treatment of water containing emulsified liquids, liquids containing alkaline pH, and/or sewage in high temperature. A embodiment of said method involves: placing said material to be treated in a tank; adding a coagulant; adding a flocculant; introducing air bubbles generated in an air flow of 400 to 800 mL of air/minute/L of water; and membrane diffusers with pore size of 1 to 2.5 mm, for a period of time sufficient to cause coagulation of at least part of the residue; introducing the air bubbles; repeating the operation for enough time to cause the flocculation of at least part of the residue, forming flakes; Introducing microbubbles with a size range of 20 to 100 µm for a period of time sufficient to cause flotation of at least part of the flakes; and removing at least part of the effluent flakes.

The document US 8,366,938, entitled "*Method and device for purifying liquid effluents*", was published on 05Fev2013. Said document reveals a method in which water is separated from other substances by air bubbling, in a vertical apparatus (3), in effluents fed at a flow rate "D". The apparatus has a free surface and includes at least two compartments (4, 5, 6, 7) which communicate with each other to provide successive circulation from top to bottom and from bottom to top, between the lower portion of the apparatus and an average level at an airflow rate at least three times greater than the effluent flow rate. The supernatant phase is continuously discharged and the chemical oxidation of the liquids or gases from said effluent is simultaneously conducted in the same apparatus. The chemical oxidation rate and bubble flow rate (and size) are selected so as to progressively obtain the separation of the solid/liquid and liquid/liquid phases on the surface of the apparatus to obtain a chemical oxygen demand (DQO) below a predetermined limit.

The document US 2013/0264281 A1, entitled "*Apparatus and Methods for Control of Waste Treatment Processes*", was published on 10Out2013. Said document reveals a process of effluent treatment which is enhanced through the production and introduction of specific microbial populations, customized to perform specific tasks during the process for formation or precipitation of certain nutrients or to reduce the formation of solids in post-treatment. The process consists essentially of removing two parts of activated sludge, returning one of them to the treatment tank and forwarding the other to another tank, where a further microbial population is produced in a controlled manner which is subsequently reintroduced into the main tank.

As can be seen, the concept of *on-site* and *ex-situ* enrichment, revealed in 2003, has had few developments to date, and there are still substantial limitations to such equipment and processes. In the present invention, instead of the term *ex-situ* microbial "enrichment", the term *ex-situ* "modulation" is used of microbial consortium, since the invention provides from the selective or total ex-situ elimination of microorganisms to the *ex-situ* improved enrichment. This combination of factors is not obtained with the approaches known in the art.

The present invention provides improvements on the equipment and processes known until then, among other technical reasons, for providing improved *on-site* and *ex-situ* equipment and process of microbial consortia modulation present in effluent treatment tanks or in open liquid bodies such as rivers, ponds, lagoons and coves. Differently from what is revealed in the background, the *on-site* and *ex-situ* equipment of the invention comprises one or more devices for generating microbubbles/nanobubbles, thin films, shearing, or combinations thereof. The equipment of the invention provides improved performance and improved microbial consortia modulation present in a main tank of an effluent treatment unit and/or of microbial consortia present in liquid bodies such as rivers, ponds, lagoons and coves. Such approach is not mentioned or even suggested in the prior art.

Based on patent and non-patent literature, it is clearly noted the need to search new alternative solutions to those already existing to overcome the limitations of biological treatment systems for solid, semi-solid and/or liquid waste, as well as the limitation of approaches of treatment of open liquid bodies such as rivers, ponds, lagoons and coves. The present patent application reveals solutions to these problems. From what can be deduced from the researched literature, no documents were found anticipating or suggesting the teachings of the present invention which, in the eyes of the inventors, has novelty and inventive activity against the prior art.

### Summary of the Invention

The present invention has as an inventive concept common to its various objects an *on-site* and *ex-situ* equipment for the microbial consortia modulation comprising one or more devices for generating microbubble, thin film, shearing, or combinations thereof. Said equipment is particularly useful improving the conditions of open liquid bodies and/or improving the performance of treatment systems of liquid, solid or semi-solid effluents.

The equipment of the invention provides: reduction of energy consumption; increase of efficiency of treatment processes or increase of efficiency of environmental treatment or recovery processes; a volume of material containing biomass with adjustment in the present microbial consortium, providing consortium adjustment of aerobic treatment tanks or open liquid bodies; use of effluents as biomass for biotransforming and obtaining products of economic interest.

It is an of the objects of the invention an equipment for microbial consortia modulation characterized in that it comprises one or more *on-site* and *ex-situ* subsystem(s) of microbial consortia modulation comprising one or more devices selected among: devices for generating microbubbles, thin films, shearing, or combinations thereof, for subsequent or concomitant introduction of all or part of the subsystem volume to a reactor/treatment main tank.

In one embodiment, said *ex-situ* subsystem additionally comprises:
- means for controlling the temperature and/or pH;
- stirring means;
- means to adjust the nutrients, or inhibit unwanted microorganisms;
and/or
- means for total, partial or controlled discharge of the volume of subsystem material to said reactor or main tank.

In one embodiment, the equipment of the invention is characterized in that it additionally comprises controlled means of liquid introduction from bypass of input flow of waste/effluents and/or of organic matter of open liquid bodies such as rivers, ponds, lagoons or coves.

In one embodiment, the equipment of invention additionally comprises means to adjust the amount of energy introduced in the reactor or main cultivation tank and/or in the *ex-situ* subsystem(s) of microbial consortia modulation, providing the reduction of energy consumption in moments of peak demand of the electric grid - to a minimum that does not compromise the level of treatment achievable by conventional systems.

It is another objective of the invention the use of equipment for microbial consortia modulation characterized in that *on-site* and *ex-situ,* for subsequent and concomitant introduction of the volume of material containing microorganisms and/or organic matter to treatment tanks of liquid, semi-solid and/or solid effluents.

In one embodiment, said use is characterized in that the *ex-situ* subsystem of microbial consortia modulation promotes the selective growth of one or more microorganisms already present in the effluent at the expense of others, to the subsequent reintroduction of said microorganisms, in high concentrations, to the original effluent, thus avoiding the introduction of exogenous microorganisms to the specific environment and avoiding or reducing the negative environmental impact or risk. This selectivity may be obtained by isolation with microbiological techniques, upon analysis of the mixture of microorganisms present in the effluent or liquid body, and specific enrichment of one or more species of organisms together or separately, for subsequent separate introduction, sequentially or together, or through the use of selective culture media.

It is still another objective of the invention a process for microbial consortia modulation characterized in that it comprises:
- a step of microbial consortia modulation in one or more *on-site* and *ex-situ* subsystem(s) comprising one or more selected devices among: devices for generating microbubbles, thin films, shearing, or combinations thereof; and
- a subsequent or concomitant step of introduction of all or part of the volume of the *ex-situ* subsystem to a reactor/treatment main tank.

These and other objectives of the invention will be immediately valued by the skilled in the art and by companies with interests in the segment, and will be described in sufficient detail for its reproduction in the following description.

### Brief Description of the Drawings

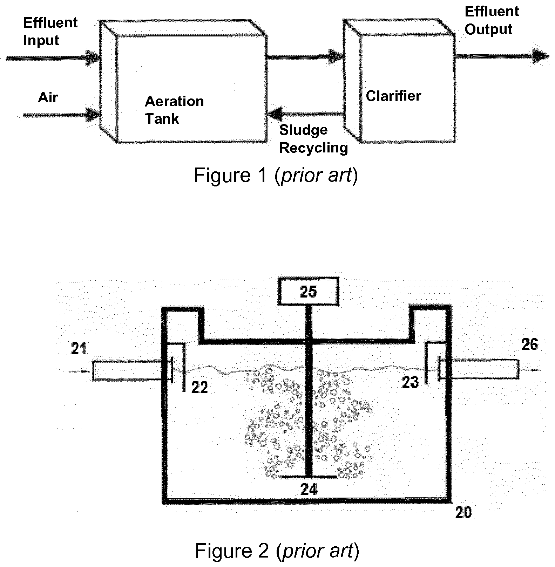

Figure 1 shows a schematic representation of a conventional process of effluent aerobic treatment (*prior art*). The enrichment of microorganisms is made with recirculation of part of the sludge after clarification.
Figure 2 shows a schematic representation of conventional equipment (20) of effluent aerobic treatment (*prior art*), being indicated: (21) effluent input; (22, 23) chicanes/deflectors; (24) blades for agitation and/or gases sprinkler; (25) engine for agitation and/or gas pump; (26) effluent output.

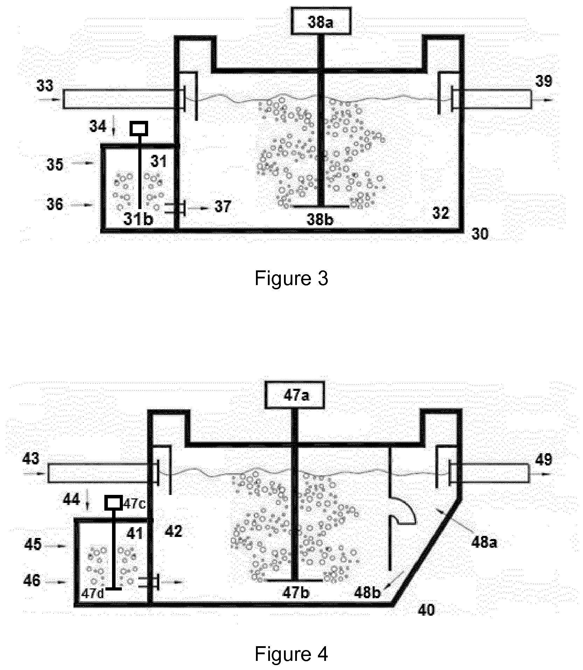

Figure 3 shows a schematic representation of an embodiment of the equipment (30) of the invention, which comprises: a main tank (32) for the treatment of liquid effluents or semi-solid waste; an *ex-situ* subsystem (31) of microbial consortia modulation comprising a device (31b) selected from: devices for generating microbubbles, thin films, shearing, or combinations thereof, for subsequent and concomitant introduction of all or part of the volume of the *ex-situ* subsystem to the main cultivation tank (32). Also indicated are: (33) entrance of effluent into main tank; (34) optional derivation of part of the input effluent for introduction in the *ex-situ* subsystem (31); (35) optional nutrient input for the *ex-situ* subsystem; (36) gas input to the *ex-situ* subsystem; (37) subsystem output of modulation of microbial consortium and feeding to the main tank (32); (38a) engine for agitation and/or gas or liquid pump with gas input; (38b) blades for agitation and/or gases sprinkler; and (39) effluent output.
Figure 4 shows a schematic representation of another embodiment of the equipment of the invention, being indicated: (40) equipment of the invention; (41) *ex-situ* subsystem of microbial consortia modulation; (42) main tank; (43) effluent input in the main tank; (44) optional derivation of part of the input effluent to feed the *ex-situ* subsystem (41) of microbial consortia modulation; (45) optional nutrient input to the *ex-situ* subsystem; (46) gases input for the *ex-situ* subsystem; (47a, 47c) engine for agitation and/or gas pump; (47b) blades for agitation and/or gas sprinkler; (47d) device selected from: devices for generating microbubbles, thin films, shearing, or combinations thereof, for subsequent and concomitant introduction of all or part of the volume of *ex-situ* subsystem to the main cultivation tank (42); (48a) subsystem or sludge decantation chamber; (48b) return of decanted sludge; (49) effluent output. This embodiment of the invention differs from similar equipment by the presence of items (41), (44), (45), (46), (47c) and (47d), that is, by the presence of *ex-situ* subsystem (41) of microbial consortia modulation.

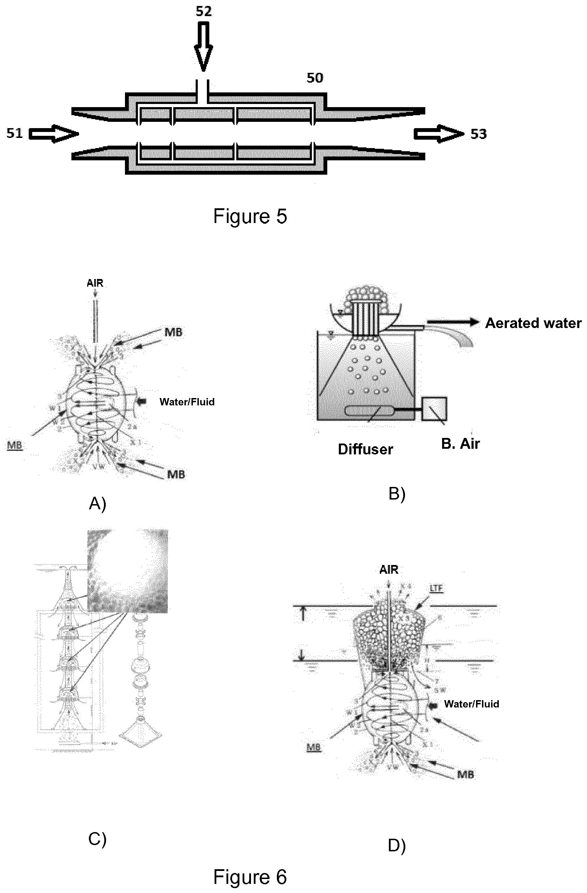

Figure 5 shows a schematic representation of a device for generation of microbubbles, nanobubbles and/or shearing. The figure illustrates a longitudinal cut of a type Venturi beak or *nozzle* (50) for the generation of microbubbles, nanobubbles and/or shearing in the liquid medium of an *ex-situ* subsystem of microbial consortia modulation, being shown in (51) the liquid input point, in (52) the gas/air input point and in (53) the output point of the liquid/microbubbles mixture. Said microbubbles and/or nanobubbles generating device can be set or adjusted to provide shearing in the liquid medium, in order to break down biofilms, aggregates or *clumps* of cells, or even break down the cell membranes of certain microbial types - or all of them.
Figure 6 shows schematic representations of devices of liquid gas dissolution known in the art. In A), it is shown a microbubbles and/or nanobubbles generating device (Riverforest Corporation) which generates microbubbles and/or nanobubbles from liquid pumping. The liquid circulation inside of said device generates a vacuum sucking the external air and the mixture with the liquid, forming the microbubbles and/or nanobubbles depending on the used flows and pressures. Liquid flows with many microbubbles and/or nanobubbles are ejected from the two ends of device. In B), it is shown a generating device of liquid thin films (Riverforest Corporation). Said device collects air bubbles in the liquid and transforms them into liquid bubbles around the air, through the forced passage by a system reducing the air bubbles rise rate (for more details, see US 8,292,271, incorporated herein by reference). The formation of liquid thin films around the air provides high gas exchange area, providing both liquid aeration and removal of gases that previously were in it (*stripping*), being therefore very useful in situations of saturation of gases in the liquid, that is, to denature the liquid. In C), it is shown another generating device of liquid thin films (Riverforest Corporation), in a vertical serial arrangement. In the detail, liquid bubbles around the air are shown. In D), it is shown a combined device, generating microbubbles and liquid thin films in the same device (Riverforest Corporation). Said microbubbles/nanobubbles generating devices may be set or adjusted to provide shearing in the liquid medium, in order to break down biofilms, aggregates or *clumps* of cells, or even break down cell membranes of certain microbial types - or all of them.

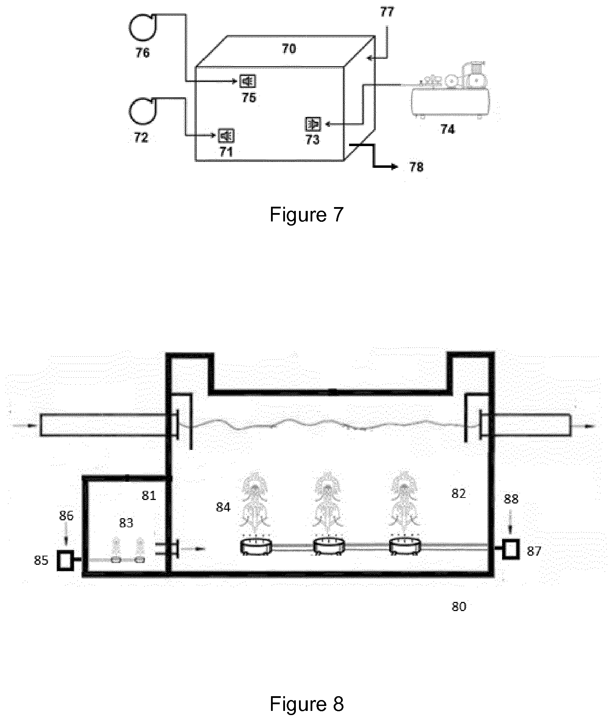

Figure 7 illustrates schematically an embodiment of equipment of the invention, in which an *ex-situ* subsystem of microbial consortia modulation (70) of 300 L comprises an arrangement of three devices, wherein:
   - a microbubbles/nanobubbles generating device (71) of BT-50 model (Riverforest Corporation) in hydraulic connection with a liquid pump (72) of 0.5 HP (WEG);
   - a liquid thin film generating device (73) of FB-50 model (Riverforest Corporation) connected to a clean air compressor (74) (Schulz); and
   - a microbubbles and liquid thin films generating device (75) of FBT-50 model (Riverforest Corporation) hydraulically connected to a liquid pump (76) of 0.5 HP (WEG). Also indicated are: (77) material input in the *ex-situ* subsystem, and may be derived from part of the volume of a main tank of treatment or derived from part of the sludge volume of a liquid body such as river, pond, lagoon or cove; (78) material output of the *ex-situ* subsystem to feed a main tank of treatment.
Figure 8 shows a schematic representation of an embodiment of the equipment (80) of the invention, comprising: a main tank (82) for treatment of liquid effluents; an *ex-situ* subsystem (81) of microbial consortia modulation comprising a device (83) devices for generating microbubbles and/or thin films FBT 50 model (Riverforest), for subsequent and concomitant introduction of all or part of the volume of the *ex-situ* subsystem to the main cultivation tank (82). Also indicated are: (84) aeration system of the main tank, comprising conventional aerators of perforated plates and thin film generator AWA (Riverforest); (85) air and/or liquid pump; (86) air and/or liquid input in the; (87) air pump for the main tank; (88) clear air input for the main tank. The other points not indicated are similar to those of Figure 3.

### Detailed Description of the Invention

The present invention has as inventive concept common to its objects an *on-site* and *ex-situ* equipment for the microbial consortia modulation comprising one or more devices for generating microbubbles, thin films, shearing, or combinations thereof. Said equipment is particularly useful in the improvement of conditions of open liquid bodies and/or in the improvement of performance of treatment systems of liquid, semi-solid or solid effluents. The equipment of the invention provides: reduction of energy consumption; increase of efficacy of processes of environmental treatment or recovery; a volume of material containing biomass with adjust in the present microbial consortium, providing consortium adjustment of tanks of aerobic treatment or open liquid bodies; use of effluents as biomass for biotransformation and obtainment of products of economic interest.

In one embodiment, the equipment of the invention comprises one or more devices for the generation of microbubbles and/or nanobubbles in the *ex-situ* modulation system, providing substantial improvement of the diffusion and solubilization of relevant gases for the cultivation of said organisms, further reaching higher titers and/or growing faster.

In one embodiment, the equipment of the invention comprises one or more devices for the generation of microbubbles and/or nanobubbles in the ex-situ modulation system, said device(s) being adjusted or adjustable to operate in differential shearing regimes, that is, to provide controlled shear force to break down biofilms, aggregates or *clumps* of cells, or to break down selectively or totally the cell membranes of microorganisms. Said device(s) thus provide(s) and additional selection medium or microbial consortia modulation present in the liquid which passes by device(s), or it is present in the main tanks of treatment or even in open liquid bodies.

In one embodiment, the equipment of the invention comprises one or more devices for the generation of liquid thin films in the ex-situ modulation system, providing substantial improvement of diffusion and solubilization of relevant gases for cultivation of said organisms, at the same time wherein they remove undesirable gases (*stripping*).

In one embodiment, the equipment of the invention comprises a combination of two or more devices selected from: devices for the generation of microbubbles/nanobubbles, with or without shearing production; and devices for the generation of liquid thin films.

The invention provides the combination of synergies of: (i) increasing the amount and/or gas dissolution rate in liquids; (ii) removing undesirable gases (*stripping*); (iii) differential shearing for selective elimination, or total shearing, for total elimination of microorganisms; (iv) reducing energy consumption; (v) adjusting the equipment or process according to variations of input or parameters load of interest.

The equipment of the invention resolves several technical problems in the sector, which are not resolved by conventional approaches of addition of dry, lyophilized microorganisms, or in the form of spores, which are cultivated *off-site*, that is, in distant locations and imply in slow adaptation of said organisms until an active rate of metabolism, as well as leading to considerable logistical difficulties.

Another problem solved by equipment and process of the invention is to provide locally a volume of the material:
(i) with microorganism(s) in virtually unlimited amounts and/or in high titers;
(ii) with selected consortia microorganisms; and/or
(iii) with only the organic matter, due to total shearing of previously existing cells in said volume,
to introduce the main tank of treatment system.

The invention avoids the acquisition, transportation and/or use of previously packaged microorganisms (notably exogenous), which reduces or eliminates the need to acquire substantial amount of microorganisms in the form of input.

In addition, the invention provides the selection and use of specific microorganisms already present in the ecological system, thereby avoiding or decreasing undesirable environmental impact or risk. This is feasible, among other forms, by the analysis of local composition of microorganisms (definition of bacteria, fungi, protozoa and other single-celled organisms) followed by selection and enrichment of desired population. The enrichment is made through microbiological techniques as: isolation and biochemical and/or genetic/genomic characterization of desired organisms in solid (plate) or liquid culture medium, or through the use of selective medium favoring the growth of desired organisms and / or the inhibition of unwanted. Other methods are also applicable as the amplification of these joint or separate organism(s), and subsequent addition thereof to the main tank together, or sequentially, for example to optimize enzymatic actions arising from the addition.

In one embodiment, the concentration of microorganisms or titer in the ex-situ modulation subsystem is at least 100 times greater than concentration of microorganisms or titer in the tank containing liquids to be treated (main tank), or water course, so that the addition of relatively small volume of wort of ex-situ modulation subsystem substantially enriches the amount and concentration of microorganisms, or titer, in said receptor body. In this embodiment, the addition of wort of ex-situ modulation subsystem to the main tank substantially modifies the total titer of microorganisms which degrade the residue or effluent in said tank without thereby requiring the addition of significant volume of wort to the main tank.

In one embodiment, the ex-situ modulation subsystem comprises a microbubbles and/or nanobubbles generating device adjusted to produce shearing intensity which provides the rupture of films or cell aggregates and/or selectively eliminates certain microorganisms due to the rupture of the cell membrane thereof. In another embodiment the shearing is total, breaking all cellular structures and converting them into acellular organic matter. In any of the latter two embodiments, the sludge present in liquid bodies as rivers, ponds, lagoons or coves can, once being submitted to ex-situ modulation subsystem, be loaded into a main tank of aerobic treatment. Similarly, in any of the latter two embodiments, a volume derived from a treatment station can be reused, once it is subjected to the ex-situ modulation subsystem, being loaded to a main tank of aerobic treatment and in it providing fragments resulting from partial or total cellular rupture in the form of nutrients and / or enzymes useful in the treatment process or conversion of organic matter into other substances of economic interest.

Such embodiments, independently of each other, provide, among other advantages: more amplitude of adjustment of microorganism consortia than conventional systems; the selection of specific microorganisms to act in the system; the selection of concentration bands of microorganisms to act on the system; the selection of the time when such a volume containing microorganisms (or organic matter without microorganisms) is added; the high speed of change of concentration of microorganism(s) in the system, which makes it possible to adjust the process according to the variations of the input material; high rate of metabolism of microorganisms in the ex-situ modulation subsystem, to degrade and/or transform the undesirable substances in the main tank; reduction of residence times; higher treatment productivity; reduction of energy consumption; reduction or elimination of unwanted or risky environmental impacts; conversion of organic matter into other substances of economic interest.

The use of the invention provides Substantial improvement in the efficiency of effluent treatment systems, providing: substantial increase of availability of microorganisms in large quantities and in a high state of metabolic activation for introduction into treatment systems; elimination or minimization of transport logistical difficulties and/or stock of microorganisms, as well as the environmental and/or human health risks arising from the transportation of microbial material in large amount; substantial reduction of energy consumption related to introduction of gases (for example, air) necessary for microbial growth; substantial increase of performance of effluent treatment systems, with the increase of capacity of units already installed and/or with the reduction of installation costs of new units; differential shearing to additional microbial consortia modulation; total shearing to convert microbial biomass into acellular organic load; to cause biotransformation or to induce or modify the organisms in the main tank; technical viabilization of effluents use as biomass for biotransformation.

In one embodiment, the availability of large quantities of microorganisms cultivated according to the present invention and in a high state of metabolic activation, for subsequent introduction in the environment where such waste is found is an interesting and promising alternative and differs from other similar approaches, as will be demonstrated in more detail below.

The present invention also provides a solution to the problem of high energy required to introducing air and/or its dissolution in the liquid body, which occurs in conventional systems, when providing equipment consisting in a reactor or main cultivation tank provided with *ex-situ* subsystem of microbial consortium modulation. The amount of energy required to obtain high titers of microorganisms in the equipment of the invention is substantially lower than that required for the cultivation of microorganisms in reactors or conventional cultivation tanks. Said equipment enables, in practice, an *on-site* process in which microbial consortia are modulated *ex-situ*, substantially reducing these and other limitations of obtaining high titers of microorganisms and can eliminate or reduce the need for reuse of activated sludge, decreasing the total amount of this sludge produced in the process.

In one embodiment, microbubbles of air and/or oxygen are introduced into the liquid medium of the ex-situ subsystem and/or to the liquid body to which organisms will be introduced, substantially increasing the rate of dissolution of the oxygen to said liquid body(bodies) and thereby reducing one of the major bottlenecks for microbial growth. The introduction of desirable gases (oxygen) and/or the removal of undesirable gases (such as methane, H₂S and others) through the use of systems of intensive introduction of microbubbles contribute in this process.

In another embodiment, microbubbles of CO₂ and/or nitrogen are introduced into the liquid medium of the ex-situ subsystem and/or to the liquid body to which the organisms are introduced, to promote anaerobic digestion and/or the production of biogas, or in process to selectively decrease or increase inorganic (e.g., ammonia, nitrites, phosphates, acids, alkalis or salts) or organic components (agricultural defenses, lipids, proteins and peptides, sugars, petroleum and its derivatives, paints and dyes or other industrial or domestic compounds), selective conditions of pH, nutrients, and inhibitors are applied to the system in order to promote the ex-situ modulation of the desired microbial consortia, and the relative or absolute decrease of unwanted microorganisms.

It is one of the objects of the invention an equipment for microbial consortia modulation characterized by comprising one or more *on-site* and *ex-situ* subsystem(s) of microbial consortia modulation comprising one or more devices selected from: devices for generating microbubbles, thin films, shearing, or combinations thereof, for subsequent and concomitant introduction of all or part of the subsystem volume to a reactor/main tank of treatment.

In one embodiment, said *ex-situ* subsystem additionally comprises:
- means for controlling the temperature and/or pH;
- stirring means;
- means to adjust the nutrients, or inhibit unwanted microorganisms;
and/or
- means for total, partial or controlled discharge of the volume of subsystem material to said reactor or main tank.

In one embodiment, the equipment of the invention is characterized in that it additionally comprises controlled means of introducing liquids from inlet flow by-pass of waste/effluents and/or from organic matter to open liquid bodies, such as rivers, ponds, lagoons or coves.

In one embodiment, the equipment of the invention e characterized in that the gas introduction means also functions as a stirring means.

In one embodiment, the equipment of the invention additionally comprises means for adjusting the amount of energy introduced into the reactor or main cultivation tank and/or in the *ex-situ* subsystem(s) of microbial consortia modulation, providing the reduction of energy consumption in moments of peak demand of the electric grid - to a minimum that does not compromise the level of treatment achievable by conventional systems.

Another objective of the invention is the use of equipment for microbial consortia modulation characterized by being *on-site* and *ex-situ*, for subsequent and concomitant introduction of the volume of material containing microorganisms and/or organic matter to treatment tanks of liquid, semi-solid and/or solid effluents.

In one embodiment, said use is characterized in that the *ex-situ* subsystem of microbial consortia modulation promotes the selective growth of one or more microorganisms already present in the effluent at the expense of others, for the subsequent reintroduction of said microorganisms, in high concentrations, to the original effluent, this avoiding the introduction of exogenous microorganisms to the specific environment and avoiding or reducing the negative environmental impact or risk.

It is yet another of the objects of the invention a process for microbial consortia modulation characterized by comprising:
- a step of microbial consortia modulation in one or more *on-site* and *ex-situ* subsystem(s) comprising one or more devices selected from: devices for generating microbubbles, thin films, shearing, or combinations thereof; and
- a subsequent or concomitant step of introduction of all or part of the volume of *ex-situ* subsystem to a reactor/main tank of treatment.

In one embodiment, the process of the invention additionally comprises the control of temperature, pH, input of nutrients or other substances and/or diffusion of gases in the *ex-situ* subsystem.

In one embodiment, the process of the invention is conducted so that the operating regime is: cultivation of microorganisms in the ex-situ subsystem; and biotransformation in the main tank. In other embodiment, the process of the invention is conducted so that the operating regime is: partial or total shearing of microorganisms in the *ex-situ* subsystem; and microbial cultivation and/or biotransformation in the main tank. In both said embodiments, the process of the invention provides for obtaining the products if economic interest from the compounds present in the effluent.

The equipment of the invention provides an efficient and advantageous alternative to the other approaches currently available for environmental applications. Figure 1 (*prior art*) illustrates one of the approaches known in prior art, consisting of a step of aerobic cultivation of microorganisms and reintroduction of sludge from the subsequent step of clarification. Through this technique approach, the titer of microorganisms in the equipment of aerobic cultivation can be increased, but it is not feasible to select specific microorganisms for introduction into said equipment.

Figure 2 (*prior art*) shows a schematic representation of equipment for the aerobic treatment of effluents equipped with engine, system of aeration and mixing, effluent input and output.

The approaches shown in Figures 1 and 2 do not provide the same technical effects as the invention. Moreover, they do not provide all the technical effects of the invention concomitantly: the selection of specific microorganisms for introduction into the treatment tank / system; the selection of concentration bands of microorganisms to act in the system; the selection of the moment at which such microorganisms are added; the high speed of change of concentration of micro-organism(s) in the system, which enables the adjustment of the process according to the variations of the input material; the reduction of residence times; higher treatment productivity; the reduction of energy consumption; modulation of the microbial consortia through shearing differential and/or total shearing; the conversion of microbial biomass into acellular organic matter.

The equipment of the invention provides much more flexibility of operation than the conventional ones and a significant magnification of the magnitude of microbial consortia modulation, since the complete elimination of living microorganisms is the elevation of the titer of microorganisms. In both cases, the equipment provides flexibility and high capacity of removal of organic load and/or conversion of the organic load in other substances of economic interest.

Said microorganisms or microbial consortia comprise or are selected from the group comprising bacteria, *Archaea*, yeasts, fungi or protozoa, or any mixture thereof.

In one embodiment, the process of the invention is conducted from the use of at least part of the volume of a liquid effluent to be treated, by bypassing in a bypass system or in a sequential system, as a source of organic matter and/or microorganisms whose consortium is modulated by the ex-situ subsystem and subsequent and concomitant introduction or reintroduction of all or part of the volume of the ex-situ subsystem, containing material enriched with microorganisms and/or acellularized organic matter, to the original liquid body.

In other embodiment, inorganic or organic compounds are added or removed from liquid effluent so as to promote specifically desired microorganisms species and/or inhibit the growth or permanence of species of unwanted microorganisms.

The process of the invention is applicable, among others, to the treatment of domestic sewage. The composition of the sanitary sewage can be exemplified in table 1 below, which also shows the approximate values of the respective components arriving per day to a treatment station of sanitary effluents from a population of 1,5 million inhabitants (considering flow rate of 2,500 L/s and sewage density of 1.0):

**Table 1 - Domestic sanitary sewage and amounts generated per day of some substances.**

| **Component/substance** | **Weight %** | **Tons/day** |
|---|---|---|
| Organic matter | 0.196 | 423 |
| Proteins | (between 40 and 60%) | 170 to 254 |
| Carbohydrates | (between 25 and 50%) | 105 to 211 |
| Fats/fatty matters | (approx. 10%) | 42 |
| Minerals | 0.004 | 8.6 |

As can be seen in table 1, the amount of organic matter which arrives daily at the treatment stations of domestic effluents is very large. The present invention provides substantial advantages and solves technical problems relevant to the treatment and/or reuse of this biomass, organic load or individual components. The equipment and/or process of the invention enables, in practice, the use of this enormous source of biomass as input, notably when the process of the invention is conducted in such a way that the operating regime is: microorganism consortia modulation in the *ex-situ* subsystem; and biotransformation in the main tank, thus enabling to obtain the products of economic interest from compounds present in the effluent. Optionally, the imposition of differential or total shearing on the ex-situ subsystem provides differential or total cell disruption, providing in the middle the contents until then intracellular in the medium, providing additional nutrients and/or specific enzymes.

The following examples are intended merely to exemplify some of the many ways of realizing the invention without, however, limiting the scope thereof.

### EXAMPLES

### Example 1. Equipment for ex-situ modulation of Microbial Consortia

Figure 3 shows a schematic representation of one embodiment of the invention, indicating an equipment (30) comprising: a main tank (32) for the treatment of liquid effluents or semi-solid waste; an *ex-situ* subsystem (31) of microbial consortia modulation comprising a device (31b) selected from: devices for generating microbubbles, thin films, shearing, or combinations thereof, for subsequent and concomitant introduction of all or part of the volume of the *ex-situ* subsystem to the main cultivation tank (32). Also indicated are: (33) effluent input in the main tank; (34) optional derivation of part of the input effluent for introducing into the *ex-situ* subsystem (31); (35) optional nutrient input for the *ex-situ* subsystem; (36) gases input for the *ex-situ* subsystem; (37) subsystem output of microbial consortium modulation and feed to the main tank (32); (38a) engine for agitation and/or gas or liquid pump with gas input; (38b) blades for agitation and/or gas sprinkler; and (39) effluent output.

The equipment of the invention provides much more flexibility of operation and a significant amplification of the modulation of microorganism consortia, either by the substantial increase of the titer of one or more microorganisms or by the selective elimination of one or more microorganisms by differential shearing, thereby increasing the removal capacity of organic load of an existing or new treatment tank. In one embodiment, the title of microorganisms in the ex-situ subsystem (31) is at least 100 times greater than the titer of microorganisms in the tank (32) containing the main volume of effluent to be treated. Depending on the operating conditions this titer can be between 1,000 and 10,000 times higher, comparing the titers in subsystem (31) and main tank (32). Accordingly, one of the advantages provided by the equipment of the invention is the fact that the addition of relatively small volumes of cultivation wort from the ex-situ subsystem (31) to the main tank (32) provides substantial enrichment of the amount and concentration of microorganisms, or titer, in said main tank. This substantial technical effect enables the design and operation of equipment in which the volume ratio between subsystem (31) and main tank (32) can be from 1:10 to 1:1,000,000. The range of proportions may be even broader, depending on the specific case and / or the operating conditions. It is noted that the amount of gas required for a volume of 100 to 10,000 times less liquid is also substantially lower. Accordingly, the amount of energy required to introduce gases into such a smaller volume is also substantially lower. Thus, obtaining high concentrations of microorganisms in the subsystem (31) of the invention is enabled with substantial reduction of energy, especially when compared to the energy required for the introduction of gases (and similar concentration of microorganisms) into conventional aerobic systems.

### Example 2. Equipment for ex-situ modulation of microbial consortia with sludge recycling

Advantages similar to those provided by the equipment of example 1 are provided by the equipment of another embodiment of the invention, schematically shown in Figure 4. The equipment (40) of this embodiment of the invention comprises: (41) *ex-situ* subsystem of microbial consortia modulation; (42) main tank; (43) effluent input in the main tank; (44) optional derivation of part of input effluent to feed the *ex-situ* subsystem (41) of microbial consortia modulation; (45) optional nutrient input for the *ex-situ* subsystem; (46) gases input for the *ex-situ* subsystem; (47a, 47c) engine for agitation and/or gas pump; (47b) blades for agitation and/or gas sprinkler; (47d) device selected from: devices for generating microbubbles, thin films, shearing, or combinations thereof, for subsequent and concomitant introduction of all or part of the volume of the *ex-situ* subsystem to the main cultivation tank (42); (48a) subsystem or sludge decantation chamber; (48b) return to decanted sludge; (49) effluent output. This embodiment of the invention differs from the similar equipment by the presence of items (41), (44), (45), (46), (47c) and (47d), that is, by the presence of *ex-situ* subsystem (41) of microbial consortia modulation.

### Example 3. Equipment for ex-situ modulation of microbial consortia with Microbubbles generator with com shearing production

In one embodiment, the equipment of the invention comprises a microbubbles generating device in the *ex-situ* subsystem, to provide differential or total shearing. Figure 5 shows a schematic representation of a device for generation of microbubbles, nanobubbles and/or shearing. The figure illustrates a longitudinal cut of a beak or *nozzle* type Venturi (50) for the generation of microbubbles, nanobubbles and/or shearing in the liquid medium of a *ex-situ* subsystem of microbial consortia modulation, being shown in (51) the liquid input point, in (52) the gases/air input point and in (53) the liquid/microbubbles mixture output point. Said microbubbles and/or nanobubbles generating device can be set or adjusted to provide shearing in liquid medium, so as to break down biofilms, aggregates or *clumps* of cells, or even break down cell membranes of certain microbial types - or all of them.

### Example 4. Process for the rupture of films of microorganisms and/or algae with differential shearing

Figure 7 illustrates schematically one embodiment of equipment of the invention, in which an *ex-situ* subsystem of microbial consortia modulation (70) of 300 L comprises an arrangement of three devices, where:
- a microbubbles/nanobubbles generating device (71) of BT-50 model (Riverforest Corporation) in hydraulic connection with a liquid pump (72) of 0.5 HP (WEG);
- a liquid thin films generating device (73) of FB-50 model (Riverforest Corporation) connected to a clean air compressor (74) (Schulz); and
- a liquid thin films and microbubbles generating device (75) of FBT-50 model (Riverforest Corporation) hydraulically connected to a liquid pump (76) of 0,5 HP (WEG). Also indicated are: (77) material input into the *ex-situ* subsystem, and can be derived from part of the volume of a main tank of treatment or can be derived from part of the volume of sludge of a liquid body such as river, pond, lagoon or cove; (78) material output from the *ex-situ* subsystem to feed a main tank of treatment.

The equipment of this embodiment of the invention was used for the rupture of films of microorganisms (Without the cell membrane rupture), to improve the performance of an effluent treatment system. 200 liters of sludge and liquids of a pond with high organic load, including films of microorganisms and algae, were introduced into said subsystem (70). Thereafter, the liquid pump (72) was triggered, hydraulically connected to a microbubbles generating device (71). In only 2 minutes of operation, all *clumps* of cells or biofilms of microorganisms or algae were no longer detectable, due to moderate shearing generated in the device (71).

### Example 5. Process for ex-situ modulation of microbial consortia - imposition of aerobic regime and odor removal

The equipment of example 4 was used for odor removal of 200 L of dirty water obtained in pond contaminated with domestic sewage. In this example, the three devices (71, 73 and 75) were driven, respectively by pumps (72, 74) and by compressor (76). Due to the great air dissolution in the *ex-situ* subsystem in this experiment, the characteristic bad smell of anaerobic systems was no longer noticeable in 90 minutes of operation. In this context, it should be noted that the invention, in this embodiment or in other embodiments thereof, provide much more flexibility of operation and significant amplification of the amount and speed of dissolution of gases in the liquid body, and accordingly, the removal capacity of organic load. On the one hand, the amount of oxygen present in the air (21% by volume, 23% by weight) and the air density (approximately 1.2 kg/m³), determine that each cubic meter of air has 276 g of O₂. On the other hand, 8.3 mg/L is the limit of oxygen saturation dissolved in fresh water in the temperature of 25°C, as shown below in table 2.

**Table 2 - Solubility of Oxygen in fresh water (without salinity)**

| | | | | | | | | | | |
|---|---|---|---|---|---|---|---|---|---|---|
| abs Pressure | *mm Hg* | 760 | | | 1520 | | | 3040 | | |
| | *psi* | 14.7 | | | 29.3 | | | 58.7 | | |
| | *bar* | 1 | | | 2 | | | 4 | | |
| | *kPa* | 101.1 | | | 202.2 | | | 404.3 | | |

| Temperature | | Solubility | | | | | | | | |
|---|---|---|---|---|---|---|---|---|---|---|
| *°C* | *°F* | *µMol* | ***mg*/*L*** | *ML*/*L* | *µMol* | *mg*/*L* | *ML*/*L* | *µMol* | *mg*/*L* | *ML*/*L* |
| 5 | 41 | 399 | 12.8 | 9.1 | 798 | 25.5 | 18.2 | 1595 | 51.1 | 36.4 |
| 10 | 50 | 353 | 11.3 | 8.2 | 705 | 22.6 | 16.4 | 1411 | 45.1 | 32.8 |
| 15 | 59 | 315 | 10.1 | 7.5 | 630 | 20.2 | 14.9 | 1260 | 40.3 | 29.8 |
| 20 | 68 | 284 | 9.1 | 6.8 | 568 | 18.2 | 13.7 | 1137 | 36.4 | 27.3 |
| 25 | 77 | 258 | **8.3** | 6.3 | 517 | 16.5 | 12.6 | 1034 | 33.1 | 25.3 |
| 30 | 86 | 236 | 7.6 | 5.9 | 473 | 15.2 | 11.8 | 947 | 30.3 | 23.6 |
| 35 | 95 | 218 | 7 | 5.5 | 436 | 14 | 11 | 872 | 27.9 | 22.1 |
| 40 | 104 | 202 | 6.5 | 5.2 | 404 | 12.9 | 10.4 | 808 | 25.9 | 20.8 |

In the limit wherein the O₂ present in the air is totally dissolved in water, each cubic meter of air totally dissolve in water represents the dissolution of 276g of oxygen. Tests performed in the lab with the equipment of this embodiment of the invention indicate an efficiency of 70% of dissolution of air with the equipment, and can be greater depending on the operating conditions. In these conditions, the equipment of the invention provides, for the injection of each cubic meter of air into the liquid, the dissolution of 193.2 g of O₂ (and 161.2 g of O₂ for the use of air with high humidity, whose density is approximately 1 kg/m³ of air).

### Example 6. Process of ex-situ Modulation of Microbial consortia for environmental applications

One embodiment of the process of the invention for the treatment of domestic sanitary sewage comprises:
- a step of microbial consortia modulation in one or more *on-site* and *ex-situ* subsystem(s) comprising one or more devices selected from: devices for generating microbubbles, thin films, shearing, or combinations thereof; and
- a subsequent or concomitant step of introduction of all or part of the volume of the *ex-situ* subsystem to a reactor/main tank of treatment.

The process of this embodiment of the invention provides many advantages: obtaining high titers of microorganisms out of main tank of sewage treatment and its subsequent addition, in high quantity and high estate of metabolic activity; And potentiates the treatment efficiency of effluents for at least five concomitant and synergistic reasons: (i) increased treatment efficiency due to the virtually choice control of the titer of microorganisms present in the tank - provided by the addition of small volumes of concentrated liquid in microorganisms (concentration 1000 x greater or more) obtained with the ex-situ subsystem; (ii) substantial part of the aeration required in conventional effluent treatment tanks is used for organism growth, which does not necessarily imply the reduction of organic load. Thus, when introducing high titers of microorganisms into treatment tanks, virtually all of the aeration introduced therein is used for the consumption and/or immediate bioconversion of biomass/organic load by highly activated microorganisms; (iii) provides the choice of consortia of microorganisms in titers and/or proportions difficult to obtain in conventional tanks; (iv) provides for the selective introduction of organisms specifically adapted to the degradation of substances of difficult degradation which occasionally enter the system. The monitoring of the effluent composition before entering the treatment tanks (or even during) enables the departure of an ex-situ subsystem from the organism appropriate to its degradation and introduction into the tank in a timely manner to avoid substantial undesirable oscillations in the tank.

It should be noted, in this context, that the residence time in treatment tanks of domestic sewage, to mention an example, is of 18 to 36h. Thus, the cultivation time in the *ex-situ* subsystem of the equipment of the invention, being substantially lower (between 4 and 8h, depending on the size and/or operating regime), enables the adoption of control measures in a timely manner to avoid the reduction of efficiency in the system and, on the contrary, increase it; (v) the system provides for the selection and promotion of the growth of microorganisms already present in the effluent and able to degrade inorganic and / or organic compounds, to the detriment of unwanted microorganisms in the effluent, and through the promotion of drastic competitive advantage, achieve the desired effect in the effluent, without introducing non-environmental organisms avoiding or minimizing environmental impacts or risks.

The skilled in the art immediately will understand that the *ex-situ* modulation of other organism(s) (alone or in consortium) may be embodied from the concept now exemplified. The use of equipment of the invention with sequential and/or in parallel *ex-situ* subsystems, for indirect control of organisms consortia in the main treatment tank, is another embodiment immediately possible for the skilled in the art from the present teachings.

### Example 7: Process of Treatment of mangrove water or polluted lake, through the selection of microorganisms of the medium itself, enrichment thereof and return to the water course

One embodiment of the invention is specifically aimed at solving a regulatory problem, which often makes it impossible to treat water courses such as lakes, ponds, rivers, canals, rivers or sea arms, bays etc. The difficulty of prohibiting the introduction of exogenous microorganisms into such bodies by their possible and/or unknown environmental impact is solved by the use of the equipment of the invention next to such bodies of water. Referring to Figure 3, it will be understood that in this embodiment, the equipment of the invention is substantially smaller, since the tank (32) has the function of homogenizing the liquid flow from the body of water entering through the input (33) as output flow (37) of the *ex-situ* subsystem (31) and discharge (39) back to the body of water. Thus, the tank (32) is substantially smaller than conventional tanks of effluent treatment systems. Depending on the water body in question, the equipment of the invention may be portable and/or installed in movable or modifiable positions. In long water bodies, such as rivers, various equipment of the invention may be used, each in a suitable operating regime for the composition characteristics and removal of undesirable compounds.

### Example 8. ex-situ Process of Microbial consortia modulation and subsequent alteration of metabolic regime

The equipment of the invention provides the introduction and diffusion or solubilization of relevant gases, or the removal of unwanted gases, promoting the selective growth of certain microorganisms over others. In one embodiment, the equipment of the invention provides the adjustment of the conditions of introduction of gases for metabolic acclimatization of the microorganisms in the cultivation system. In a process context of the invention, microorganisms are cultivated in the ex situ subsystem (31, 41) under aeration conditions and high speed of cell multiplication, for subsequent introduction of the microorganisms so cultivated into the main tank (32, 42) operated under conditions of little or no aeration, or even in anaerobiosis. This process is particularly suitable for the use of facultative microorganisms, such as certain yeasts. The capabilities of the equipment of the invention thus enable a biotechnological process with both microbial enrichment conditions and metabolic control (which might otherwise be mutually exclusive) in a single equipment/process. The equipment of the invention also provides for adjustment of the levels/contents of relevant gases in the liquid system, so that the difference between the gas contents in the ex-situ subsystem (31, 41) and the main tank (32, 42) is optimized for better microbial performance. In one embodiment, the operating regime is the cultivation of microorganisms in the ex-situ subsystem and biotransformation in the main tank. Thus, the process of the invention is particularly useful for the conversion of the compounds in the effluent into other utility products, such as biogas, fuel, fertilizer, animal feed or raw materials for other processes.

### Example 9. Equipment and Process for the Treatment of Effluents with ex-situ modulation of microbial consortium - modulation also in the main tank with energy economy and increase in the dissolution of oxygen

In this embodiment of the invention, schematically illustrated in Figure 8, the equipment of the invention additionally comprises a thin film generating device in the main tank, so as to operate cooperatively with the devices of the ex-situ subsystem and provide increased rate of oxygen dissolution in the main tank. The apparatus (80) of this embodiment of the invention comprises: a main tank (82) for treatment of liquid effluents; an ex-situ subsystem (81) of microbial consortia modulation comprising a device (83) devices for generating microbubbles and / or thin films FBT 50 model (Riverforest), for subsequent and concomitant introduction of all or part of the volume of the *ex-situ* subsystem to the main cultivation tank (82). Also indicated are: (84) aeration system of main tank, comprising conventional perforated plate aerators and thin film generators of AWA model (Riverforest); (85) air and/or liquid pump; (86) air and/or liquid input; (87) air pump for the main tank; (88) clear air input for the main tank. This equipment, in addition to providing ex-situ means of modulation of the microbial consortia in the system, presents improved aerobic treatment performance in the main tank, with reduction of energy consumption for aeration of the main tank between 20 and 50% to obtain the same amount of oxygen effectively dissolved in the tank.

### Example 10. Energy economy in the Treatment of Effluents

A treatment system of domestic effluent equipped with an equipment and/or *on-site* process of *ex-situ* modulation of microbial consortia, according to the present invention, is particularly useful for energy efficient operation. In addition to reducing the amount of energy required for the same treatment level of effluents, said equipment and process provide a reduction in the residence time required to obtain the same treatment/removal of organic load as conventional systems.

Consequently, the equipment and the process of the invention can be operated with adjustment in the amount and energy used in the treatment system, synchronously or anti-synchronously to the cycles of energy availability in the electrical system that supplies the treatment plant. In this context, since the average residence time of the liquid in the equipment of the invention is less than the residence time of conventional systems, the process of the invention allows greater flexibility in the adjustment of the operation according to the cycle time of energy demand in the electrical system where the treatment system is connected - since the means for adjusting the amount of energy introduced into the treatment system substantially changes the costs or energy risks of operating both the treatment plant and the electrical system in which it is connected.

The application of the inventive concept of present invention additionally provides, therefore, adjustment of the use of energy in the equipment of the invention so that the moments of greater energy consumption of the equipment occur in moments of greater availability or lower cost of energy offered by the system operator.

From the point of view of the system operator, the invention is particularly useful for reducing the amount of energy consumed by the treatment systems, which can be increased at times of peak demand, increasing safety and reducing the risks of system failures.

The use of the invention also provides the reduction of energy consumption in peak moments, because the equipment of the invention can have energy consumption reduced in such moments to a minimum that does not compromise the level of treatment achievable by conventional systems.

These technical effects of the invention are highly relevant and difficult to obtain by conventional systems, bringing substantial advantages: from the point of view of the electrical system operator, the use of the invention increases the efficiency of the electrical system as a whole, notably in electrical systems based on hydropower plants, in which the energy produced is not stored, at least not adequately or substantially.

The invention thus contributes to reduce the energy consumption. In addition, it also provides reduction, selective in time, of the impact of its energy consumption on electrical systems, which can thus be operated with less variation of demand at critical moments - as is the case of typical peaks of demand that are criterion of sizing of energy systems is inferior to that observed without the use of the invention.

Those skilled in the art will appreciate the knowledge presented herein and may reproduce the invention in the embodiments presented and in other variants, falling within the scope of the appended claims.

## Claims

1. Equipment for microbial consortia modulation for environmental applications comprising:
- a reactor/main tank of treatment; and
- one or more *on-site* and *ex-situ* subsystem(s) of microbial consortia modulation **characterized in that** it comprises one or more devices selected from: devices for generating microbubbles/nanobubbles, thin films, shearing or combinations thereof.

2. Equipment according to claim 1, **characterized in that** said *ex-situ* subsystem additionally comprises:
- means for controlling the temperature and/or pH;
- stirring means;
- means to adjust the nutrients, or inhibit unwanted microorganisms; and/or
- means for total, partial or controlled discharge of the volume of subsystem material to said reactor or main tank.

3. Equipment according to claim 1 or 2, **characterized in that** it additionally comprises controlled means of liquids introduction from bypass of input flow of waste/effluents and/or of organic matter of open liquid bodies.

4. Equipment according to any one of claims 1-3, **characterized in that** it additionally comprises means to adjust the amount of energy introduced in the reactor or main cultivation tank and/or in the *ex-situ* subsystem(s) of microbial consortia modulation.

5. Equipment according to any one of claims 1-4, **characterized in that** the main tank also comprises one or more devices selected from: devices for generating microbubbles/nanobubbles, thin films, shearing, or combinations thereof.

6. Use of *ex-situ* subsystem for microbial consortia modulation, **characterized in that** it is for subsequent and concomitant introduction of the volume of material containing microorganisms and/or organic matter into treatment tanks of effluents and/or liquid bodies.

7. Use according to claim 6, **characterized in that** the *ex-situ* subsystem of microbial consortia modulation promotes the selective growth of one or more microorganisms already present in the effluent at the expense of others, for the subsequent reintroduction of said microorganisms, in high concentrations, into treatment tanks of effluents and/or liquid bodies.

8. Process for *ex-situ* microbial consortia modulation, **characterized in that** it comprises:
- a step of microbial consortia modulation in one or more *on-site* and *ex-situ* subsystem(s) comprising one or more devices selected from: devices for generating microbubbles, thin films, shearing, or combinations thereof; and
- a subsequent or concomitant step of introduction of all or part of the volume of the *ex-situ* subsystem to a reactor/main tank of treatment and/or liquid bodies.

9. Process according to claim 8, **characterized in that** the operation regime is of microorganisms cultivation in the *ex-situ* subsystem and of biotransformation in the main tank.

10. Process according to claim 8, **characterized in that** the operation regime is of differential or total shearing in the *ex-situ* subsystem and of cultivation and/or biotransformation in the main tank.

11. Process according to any one of claims 8-10, **characterized in that** it comprises a step of controlled introduction, to said equipment, of liquids from bypass of input flow of waste or effluents.

12. Process according to any one of claims 8-11, **characterized in that** it comprises differential or total shearing.

13. Process according to any one of claims 8-12, **characterized in that** it comprises the use of a conform equipment defined in any one of claims 1-5.

14. Process according to any one of claims 8-13, **characterized in that** it comprises the use of sludge of liquid bodies as input material in the *ex-situ* subsystem.
